Europäisches Patentamt

**European Patent Office**

Office européen des brevets

(11) Publication number: **0 226 671 B1**

# EUROPEAN PATENT SPECIFICATION

(45) Date of publication of patent specification: **03.04.91**  (51) Int. Cl.⁵: **A61K 9/34**, C09D 17/00

(21) Application number: **85308939.9**

(22) Date of filing: **09.12.85**

(54) Titanium dioxide suspension for use in film coating.

(43) Date of publication of application:
**01.07.87 Bulletin 87/27**

(45) Publication of the grant of the patent:
**03.04.91 Bulletin 91/14**

(84) Designated Contracting States:
**AT BE CH DE FR GB IT LI LU NL**

(56) References cited:
**CA-A- 935 255**
**DE-A- 3 004 442**
**US-A- 3 663 284**
**US-A- 3 981 984**

(73) Proprietor: **CROMPTON & KNOWLES CORPO-RATION**
**345 Park Avenue**
**New York New York 10022(US)**

(72) Inventor: **Den Boer, Patrick**
**29 Thurston Terrace**
**Glen Rock New Jersey(US)**
Inventor: **Heinze, Richard**
**284 White Oak Ridge Road**
**Bridgewater New Jersey(US)**

(74) Representative: **Hardisty, David Robert et al**
**BOULT, WADE & TENNANT 27 Furnival Street**
**London EC4A IPQ(GB)**

## Description

The present invention relates to the use of a titanium dioxide pigment suspension, in the production of a film-forming suspension.

Pigment suspensions can be used for producing a film coating on such items as pharmaceutical or veterinary tablets and confectionary pieces. The pigment suspension is typically stirred into a larger volume of polymer solution. The resulting film-forming suspension is used in the coating process. The film coating, in the form of a very thin film, should be uniform and consistent from one batch of tablets to the next.

The technique of film coating is generally known in the prior art. U.S Patent No. 2,954,323 to Endicott et al. discloses the increased efficiency and superior coating properties obtained with film coating in general as compared to other processes of coating.

The present invention relates to the use of a film-forming suspension comprising as a pigment composition titanium dioxide, water and as a dispersing agent xanthan gum, for coating pharmaceutical or veterinary tablets, or confectionery pieces or other foodstuffs. Such pigment suspensions for use in film coating are preferably sold having a concentration of titanium dioxide as high as possible. However, as the concentration of titanium dioxide increases, the suspension tends to become more viscous and may reach a point where it becomes difficult to pour from its container. Upon aging, a thick suspension of titanium dioxide may harden to the extent of becoming unusable.

In developing a high concentration pigment suspension, it is desirable to obtain a product in which the titanium dioxide particles form a stable suspension and will not settle for a prolonged period of time. The need is for a pigment suspension which will readily pour from its container and will maintain its uniform properties, during both transportation and storage, until ready for application in film coating.

U.S Patent No. 3,981,984 to Signorino discloses a pigment suspension which claims to achieve a high concentration of titanium dioxide in a non-aqueous solvent. This pigment suspension consists of titanium dioxide particles, a protective colloid such as hydroxypropyl cellulose, and a non-aqueous solvent such as ethanol. Signorino teaches that as the titanium dioxide particles are added to the solvent, the mixture becomes too viscous, and the further addition of the protective colloid serves to suspend the particles and reduce the viscosity.

In view of the increasingly strict requirements of governmental regulations in regard to the use of organic solvents, it has become desirable to obtain aqueous pigment suspensions. Canadian patent No. 935255 described a pumpable, high-solids $TiO_2$ pigment slurry in which the $TiO_2$ pigment is uniformly dispersed in water with a $TiO_2$ concentration of between 50 and 80% by weight. The slurry contains dispersing agents which are di-polar compounds of high molecular weight such as, for example, polyphosphates or amines and also a thickening agent to further inhibit settling and add the desired viscosity characteristics for pumping. These slurries are designed to be used in paper and emulsion paints.

Another aqueous titanium dioxide pigment suspension suitable for use in paper and paints is described in US-A-3663284. The $TiO_2$ suspension is between about 40 to about 80% by weight $TiO_2$ and contains at least two agents to retard settling, the first being citric or tartaric acid or a salt thereof and the second being a polysaccharide gum.

Despite these examples, however, a high content of titanium dioxide in water is not normally possible for use in film-coating. Although titanium dioxide suspensions in an aqueous sugar syrup are known, such suspensions are not generally suitable for use in a film-forming polymer solution.

The present invention involved a search for a combination of ingredients which would permit a high content of titanium dioxide particles in an aqueous suspension useful in film coating. Due to the fact that the composition may comprise merely water and a very small amount of xanthan gum, not requiring the presence of organic solvents, the composition is very simple, safe and inexpensive to make.

Thus, the present invention provides the use of a pigment composition comprising titanium dioxide, water and as a sole dispersing agent, xanthan gum, in the production of a film forming suspension for coating pharmaceutical or veterinary tablets and confectionery pieces or other foodstuffs.

The present invention is further described in the following detailed description of preferred embodiments of the invention.

The titanium dioxide pigment employed in the present invention is preferably water dispersable titanium dioxide 3328, sold by Whitaker, Clarke and Daniels of South Plainfield, New Jersey. The pigment is suitably present in an amount by weight of about 20 to 75 percent, and most preferably in an amount by weight of about 30 to 60 percent.

Titanium dioxide is a relatively heavy pigment which, when mixed in a solvent, tends to settle out and form a thick non-pourable layer of pigment on the bottom of the container. It has now been found that an excellent aqueous pigment dispersion can be obtained by the addition of a very small amount of xanthan gum.

Xanthan gum is a high molecular weight polysaccharride produced in a fermentation process by the microorganism Xanthomonas campestris. The gum, which is produced as an exocellular coating surrounding the cell wall of the microorganism, is unique and very specific, and the properties thereof are constant and reproducable under given conditions.

Xanthan gum is known as a suspending or dispersing agent in various applications. For example, xanthan gum has been used to suspend solids in ceramic glazes, paints, and textile print pastes.

The use of xanthan gum to create a high concentration titanium dioxide pigment suspension for use in a film-forming process in the food and drug industry is believed to be entirely new.

A commercially available xanthan gum, suitable in the present invention, is KELTROL®, and especially Keltrol F, a finely meshed xanthan gum, manufactured by Kelco, a division of Merk & Co., Incorporated.

The suspension of the present invention differs from other suspensions in that it can exhibit gel like behavior or very fluid behavior. Typically, the suspension actually sets up and only breaks down into a liquid by shearing action, such as produced by merely shaking the container of the pigment suspension, resulting a readily pourable pigment suspension.

The xanthan gum is generally present in the invention in amounts, by weight, ranging from 0.005 to 5 percent. As will be evident, typically only relatively very small quantities of the xanthan gum need be present in the suspension. A preferred range is from 0.05 to 0.5 percent.

Compositions of the present invention were tested by what is referred to as an oven test. An oven test is an accelerated method of assessing the long-term properties of a pigment suspension. The oven test typically involved heating the pigment suspension at $40°C$ ($104°F$)for a period, initially of 96 hours. This accelerated test is believed to be equivalent to 3 to 4 months at $29.4°C$ ($85°F$). Compositions of the present invention have withstood heating at $40°C$ ($104°F$) for one month. The oven test results were evaluated according to the following system.

RATING SYSTEM

| | |
|---|---|
| 1.0 | A rock hard or very hard settle is obtained. The suspension fails to redisperse. |
| 2.0 | A paste or semi-hard solid is obtained. The suspension fails to pour from its container without force or requires the use of a spatula. |
| 3.0 | A threshold suspension, with some supernatant, but stable. After agitation, the suspension is still thick, but pourable. |
| 4.0 | A suspension with or without supernatant but no settle is obtained. The consistency is like thick yogurt. On agitation the suspension become fluid. |
| 5.0 | A soft, fluid dispersion with no settle is obtained. It pours from its container with no agitation and flows freely. |
| 5.5 | The suspension has no settle, but is very watery. |
| 6.0 | The suspension is too watery, and is not acceptable. |

Example 1

In a blender, the following components were weighed out and mixed:

| Component | Percent by Weight |
|---|---|
| Water | 69.80% |
| Xanthan Gum | 0.20% |
| TiO$_2$ | 30.00% |

The xanthan gum was added to the water while mixing at a moderate speed. Mixing was maintained for about 3 minutes or until all of the gum had dissolved. The titanium dioxide was added slowly while the blender was mixing. The speed was adjusted to maintain a vortex in the mixture.

An oven test was performed at $40°C$ ($104°F$) for 96 hours. A rating of 5.5 was obtained. After a further period of 21 days at $40°C$ ($104°F$), a rating of 5.5 was obtained, indicating that the properties of the pigment

3

suspension remained stable.

Xanthan gum has unique properties which permit the creation of a stable and pourable titanium dioxide pigment suspension. Other gums or colloids, natural and synthetic, do not produce a satisfactory product. The following table illustrates other gums which were tried, but found unacceptable.

## Table A

### Percent Weight

| Trial/<br>Component | 1 | 2 | 3 |
|---|---|---|---|
| Distilled Water | 49.80 | 49.80 | 49.80 |
| Titanium dioxide | 50.00 | 50.00 | 50.00 |
| Guar gum | 0.20 | | |
| Polyvinylpyrrolidone | | 0.20 | |
| KLUCEL® | | | 0.20 |
| Rating | 1.0 | 1.0 | 1.0 |

As shown in Table A guar gum, polyvinylpyrrolidone, and KLUCEL®, a brand of hydroxypropyl cellulose manufactured by Hercules Co. in Wilmington, Delaware, were unacceptable, resulting in a suspension that was immediately unusable. Gum arabic was satisfactory only at high levels such as 15 percent, too high for commercial practicability.

Conventional additives may be included in the present composition, as will be understood by those skilled in the art. For example, about 0.1 percent of an antimicrobial agent such a methylpropyl paraben or potassium sorbate is suitable.

## Claims

1. The use of a film-forming suspension comprising as a pigment composition titanium dioxide, water and as a dispersing agent xanthan gum, for coating pharmaceutical or veterinary tablets, or confectionery pieces or other foodstuffs.

2. The use as claimed in claim 1 wherein the titanium dioxide is present at about 20 to 75 percent by weight, the xanthan gum at about 0.005 to 5 percent by weight the remainder of the composition being water.

3. The use as claimed in claim 1 wherein the titanium dioxide is present at about 30 to 60 percent by weight, preferably about 50 percent, and the xanthan gum from about 0.05 to 1 percent by weight, preferably from 0.02 to 0.5 percent.

4. The use as claimed in any one of claims 1 to 3 wherein the pigment composition further comprises an antimicrobial agent.

5. The use as claimed in claim 4 wherein the antimicrobial agent is methylpropyl paraben or potassium sorbate.

6. The use as claimed in claim 4 or claim 5 wherein the antimicrobial agent is present at about 0.1 percent.

7. The use as claimed in any one of Claims 1 to 6 wherein the pigment composition is admixed with a polymer solution to form the film-forming suspension.

8. The use as claimed in claim 1 wherein the pigment composition is produced by mixing together xanthan gum and water, and then adding titanium dioxide, wherein the amounts of xanthan gum, water and titanuim dioxide may be as further defined in claim 2 or claim 3.

## Revendications

1. Emploi d'une suspension filmogène comprenant comme composition de pigment du dioxyde de titane, de l'eau et comme dispersant de la gomme de xanthanne pour enrober des comprimés pharmaceutiques ou vétérinaires ou des friandises ou d'autres denrées alimentaires.

2. Emploi selon la revendication 1, dans lequel le dioxyde de titane est présent à raison d'environ 20 à 75 % en poids, la gomme de xanthanne à raison d'environ 0,005 à 5 % en poids, le reste de la composition étant de l'eau.

3. Emploi selon la revendication 1, dans lequel le dioxyde de titane est présent à raison d'environ 30 à 60 % en poids, de préférence environ 50 % en poids, et la gomme de xanthanne à raison d'environ 0,05 à 1 % en poids, de préférence de 0,02 à 0,5%.

4. Emploi selon l'une quelconque des revendications 1 à 3, dans lequel la composition de pigment comprend en outre un agent antimicrobien.

5. Emploi selon la revendication 4, dans lequel l'agent antimicrobien est du parahydroxybenzoate de méthylpropyle ou du sorbate de potassium.

6. Emploi selon la revendication 4 ou la revendication 5, dans lequel l'agent antimicrobien est présent à raison d'environ 0,1%.

7. Emploi selon l'une quelconque des revendications 1 à 6, dans lequel la composition de pigment est mélangée à une solution polymère pour former la suspension filmogène.

8. Emploi selon la revendication 1, dans lequel la composition de pigment est produite en mélangeant de la gomme de xanthanne et de l'eau et en ajoutant ensuite du dioxyde de titane, dans lequel les quantités de gomme de xanthanne, d'eau et de dioxyde de titane peuvent être telles que définies plus précisément dans la revendication 2 ou la revendication 3.

## Ansprüche

1. Verwendung einer filmbildenden Suspension, die als Pigmentzusammensetzung Titatdioxid, Wasser und Xanthangummi als Dispergiermittel enthält, für das Überziehen von pharmazeutischen oder tiermedizinischen Tabletten, Süßwarenstücken oder anderen Lebensmitteln.

2. Verwendung nach Anspruch 1, wobei das Titandioxid mit ca. 20 bis 75 Gew.-%, das Xanthangummi mit ca. 0,005 bis 2 Gew.-% vorhanden ist und der Rest der Zusammensetzung Wasser ist

3. Verwendung nach Anspruch 1, wobei das Titandioxid mit ca. 30 bis 60 Gew.-%, vorzugsweise 50 % und Xanthangummi mit ca. 0,05 bis 1 Gew.-%, vorzugsweise 0,02 bis 0,5 %, vorhanden ist.

4. Verwendung nach den Ansprüchen 1 bis 3, wobei die Pigmentzusammensetzung weiter ein antimikrobielles Mittel umfaßt.

5. Verwendung nach Anspruch 4, wobei das antimikrobielle Mittel Methylpropylparaben oder Kalziumsorbat ist.

6. Verwendung nach den Ansprüchen 4 oder 5, wobei das antimikrobielle Agens zu ca. 0,1 % vorhanden ist.

7. Verwendung nach den Ansprüchen 1 bis 6, wobei der Pigmentzusammensetzung eine Polymerlösung zugemischt ist, um die filmbildende Suspension zu bilden.

8. Verwendung nach Anspruch 1, wobei die Pigmentzusammensetzung hergestellt wird durch Zusammenmischen von Xanthangummi und Wasser und anschließende Zugabe von Titandioxid, wobei die Mengen von Xanthangummi, Wasser und Titandioxid wie in den Ansprüchen 2 und 3 angegeben betragen können.